# EUROPEAN PATENT SPECIFICATION

(11) **EP 2 922 405 B1**
(45) Date of publication and mention of the grant of the patent: **09.08.2017**
(21) Application number: 13810885.7
(22) Date of filing: 19.11.2013
(51) Int. Cl.: A22B 5/00, A22C 17/00, G01N 33/12, G01N 23/04, G02B 27/01, G01N 27/82

(54) **A METHOD FOR CONTROLLING AND ALLOWING REMOVAL OF FOREIGN MATTER FROM FOOD PRODUCTS**
VERFAHREN ZUR STEUERUNG UND AUSSCHEIDUNG VON FREMDSTOFFEN AUS NAHRUNGSMITTELN
PROCÉDÉ DE CONTRÔLE ET D'ÉLIMINATION DE MATIÈRES ÉTRANGÈRES DANS DES PRODUITS ALIMENTAIRES

(30) Priority: 22.11.2012 DK 201200738
(43) Date of publication of application: 30.09.2015
(73) Proprietor: Frontmatec Tandslet A/S, 6470 Sydals (DK)
(72) Inventor: DOHLMANN, Peter, 6470 Sydals (DK)
(74) Representative: Dalkiær, Mikkel
(86) International application number: PCT/DK2013/000080
(87) International publication number: WO 2014/079448

(56) References cited:
- WO-A1-2008/102148
- WO-A2-02/33681
- GB-A- 2 362 710
- US-B1- 6 198 834
- US-B1- 6 608 884

## Description

### The prior art

The invention relates to a method of controlling and allowing removal of undesirable parts of a food item, such as of meat, poultry, fish and vegetables, which item may be advanced on a conveyor belt, where it is currently transilluminated when passing through a non-destructive testing system by transillumination with X-rays, gamma rays or magnetic rays with a view to detecting undesirable foreign bodies, if any, in the form of bone residues, needle parts, etc., following which the item is passed to a workstation where the foreign bodies may be removed, as the location of the foreign body or bodies, if any, may be displayed on a screen to an operator.

Methods of this type are generally known, and, as regards meat, the method is performed at the slaughterhouses.

Systems and methods of this type are described in the patent literature, such as GB 2 362 710 A. The system discloses a method of controlling and allowing removal of undesired parts of a food item, such as of metal, poultry, fish and vegetables, which item may be advanced on a conveyor belt where it is currently transilluminated when passing through a non-destructive testing system by illumination with X-rays, gamma rays or magnetic rays with a view to detecting undesired foreign bodies, if any, in the form of bone residues needle parts, etc., following which the item is passed on to a workstation where the foreign bodies may be removed as the location of the foreign body or bodies, if any, is indicated to an operator on a screen.

This system, however, is vitiated by the drawback that the screen image is not very visible because of the normal work light at the cutting station, which is relatively strong. This may make it difficult for the operator to detect foreign bodies, if any, since the indication on the piece of meat is not precisely indicated.

### The object of the invention

It is the object of the invention to remedy this defect, where scanning, transillumination from a radiation source, such as an X-ray tube, forms part of the method of the slaughter line, and where the result of the scanning appears on a screen which the operator carries in front of this eyes, so that the operator may perform an accurate removal of the object, as it is indicated to him where the foreign body is precisely located in the item.

Thus, by allowing the screen image from the transillumination to be projected on a screen which the operator carries in front of his eyes, it will be possible to bring the screen image into agreement with the item so as to indicate precisely where foreign bodies, if any, are located in the item, even if the work field is strongly illuminated.

The invention ensures by simple means that, during his work, the operator can remove, i.e. cut away the foreign body, if any, more precisely and thereby with as little waste of meat as possible, as the operator, on his screen in front of his eyes, can move his head so that the screen image is visually brought into agreement with the item, which allows a more precise indication of the location in the item, even where the item is strongly illuminated.

Then, the localization of the foreign body is easy, and a cutting-away may take place in a rapid and safe manner.

Removal of needle parts, in particular, poses great problems, since they may injure the hands, but the risk is reduced in that the operator can now get an indication of the orientation and location of the needle, thereby avoiding getting into contact with the tips during the cutting-up.

It will thereby be possible to achieve a safe and gentle cutting-away with as little waste of meat as possible,

When, as stated in claim 2, the method with the projected screen image is included in known slaughter lines, a considerable degree of safety and optimum utilization of the meat will be achieved in a simple manner, as the waste is minimized.

When the wavelength of the radiation source is controlled suitably, the depth of the location of the foreign body in the item may be recorded on the screen image, thus resulting in a considerable certainty of correct cutting-away without waste and injuries.

When other food products, such as fish, vegetables and fruit, are sorted and controlled, the control of foreign bodies, such as bones and stones, will be both easier to detect and easier to remove.

Finally, as stated in claim 3, it is expedient to construct the screen as a pivotally mounted screen for a headgear article, as tilting of the screen allows convenient adjustment of the screen image relative to the least tiring position of the head relative to the work.

### The drawing

Examples of the method according to the invention will be described more fully below with reference to the drawing, in which
- fig. 1: shows an example of a known slaughter line with instructions for the operator via a screen image,
- fig. 2: shows the invention with screen image projected on a portable screen, and
- fig. 3: shows a portable screen for use in the system of fig. 2.

### Detailed description of the invention

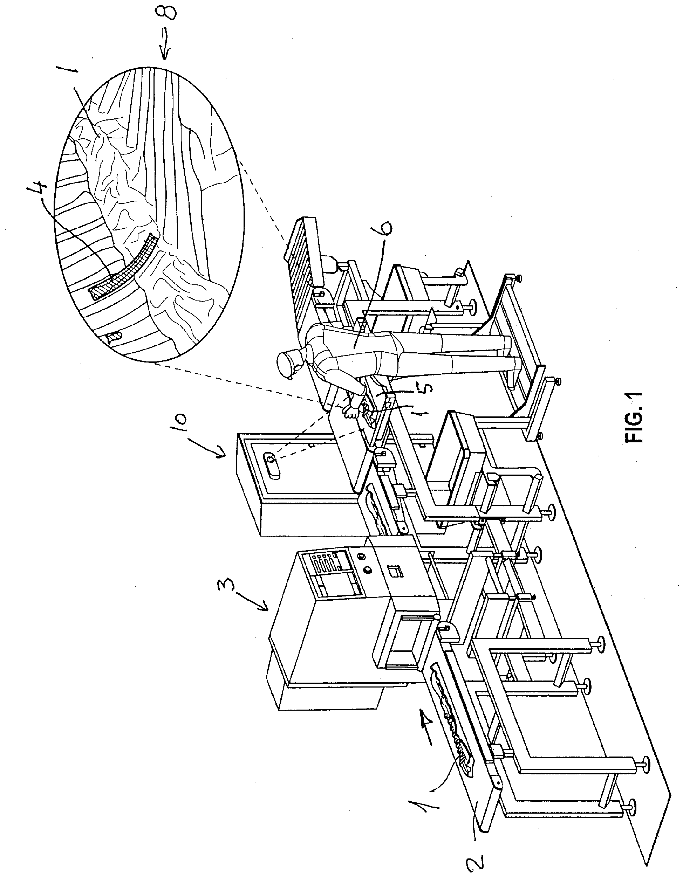

Fig. 1 shows an example of a generally known cutting-up line, as known from a slaughterhouse.

The system its built on the basis of a conveyor belt 2, which continuously advances items 1, such as pieces of meat, for further processing.

The system comprises an X-ray scanner 3, which serves to transilluminate the item 1 and thereby reveal foreign bodies, if any, concealed in the item. This may be needle residues, bone pieces, splinters etc.

The X-ray scanner 3 transmits the signal to a projector 10, which is located in front of an operator 6, said projector emitting a screen image 8 directly to the surface of the piece of meat 1, so that the operator thereby receives an indication of the presence of foreign bodies, if any, with a view to cutting these away.

However, this indication of foreign bodies 4, if any, on the piece of meat 1 is difficult for the operator 6 to see because of the relatively strong work light, which is normally present at the cutting-up stations. Of course, this applies to small foreign bodies which will not be visible because of the limited contrast indication of the foreign body on the surface.

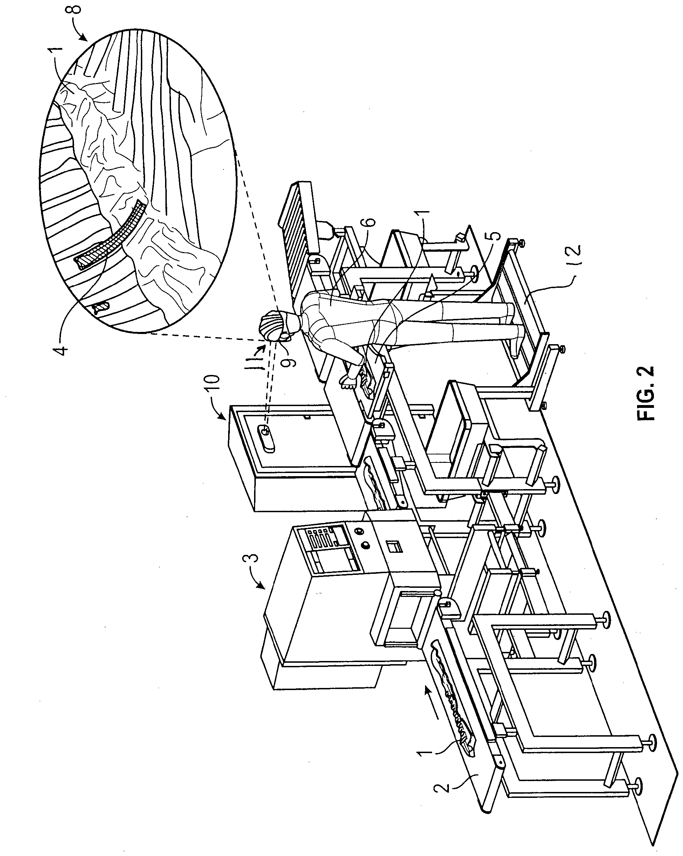

When, according to the invention, and as shown in fig. 2, the operator 6 is provided with a screen 9 in front of his eyes, the screen image 8 may be projected onto the screen 9, so that the operator 6 will be able to bring about agreement between the screen image and the item 1 itself, even though the work station, the cutting field, is strongly illuminated.

To achieve a precise image on the screen 9 of the operator 6, it is expedient that he stands on a height-adjustable base 12, it being thereby possible to adjust the base 12 to the operator's eye level, thus ensuring that the image 8 emitted from the projector 10 is applied precisely to the screen 9.

This gives the operator 6 a direct picture of where the foreign body 4 is located in the item 1, as its location is displayed directly to the operator on the upper side of the item 1.

In general, it is possible to cut away with a minimum waste of meat, just as the risk of injuries to the hand is minimized, since the operator can now observe the orientation of the needle tip precisely in the meat.

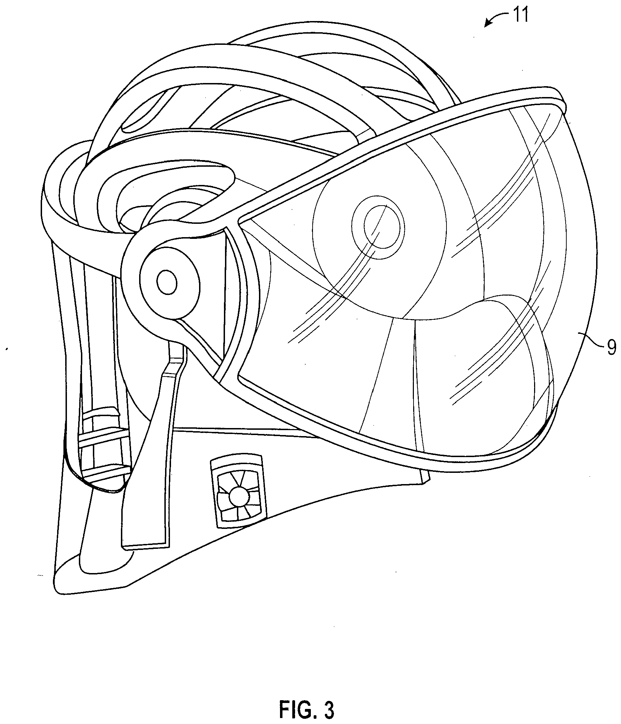

An example of a screen for use for the operator is shown in fig. 3. This comprises protection helmets, hair-covering garments 11, which are provided with a pivotally mounted screen 9 at the front.

Thus, the screen 9 may be tilted to a position convenient to the operator relative to his eyes and the position of the item 1 on the cutting table 5.

Hereby, it is easier for the operator to adjust the screen image 8, so that there will be exact agreement with the item 1.

This gives a more convenient and less tiring working posture, since the neck muscles are not strained unduly.

In the method according to the invention, it will be possible to record by suitable adjustment of the wavelength in the X-ray scanner 3 how deep the foreign body or bodies 4, if any, are located in the items 1. Moreover, foreign bodies, if any, may be accentuated by coloured light. This will facilitate the work for the operator, since he will be led directly to the desired area, and the risk of overlooking a foreign body, if any, will be reduced.

## Claims

1. A method of controlling and allowing removal of undesired parts of a food item, such as of meat, poultry, fish and vegetables, which item may be advanced on a conveyor belt, where it is currently transilluminated when passing through a non-destructive testing system by illumination with X-rays, gamma rays or magnetic rays, with a view to detecting undesired foreign bodies, if any, in the form of bone residues, needle parts, etc, following which the item is passed on to a workstation where the foreign bodies may be removed, as the location of the foreign body or bodies, if any, may be indicated to an operator on a screen, **characterized In that** the screen image (8) from the transillumination is projected on a screen (9) which the operator (6) carries in front of his eyes, so that the screen image (8) may be brought into agreement with the item (1), thereby indicating precisely where foreign bodies (4), if any, are located in the item (1).

2. A method according to claim 1, **characterized In that** the equipment (10) for the projection of the screen image (8) and the method of controlling are included in known slaughter lines as part of the system and the cutting-out.

3. A method according to claims 1 and 2, **charactorized in** that the screen (8) is pivotally mounted on a portable head garment (11) for adjusting the screen image (8) relative to the head position of the operator (6).

## Patentansprüche

1. Verfahren zum Steuern und Ermöglichen des Entfernens von unerwünschten Teilen eines Nahrungsmittelartikels, wie z.B. aus Fleisch, Geflügel, Fisch und Gemüse, wobei der Artikel auf einem Förderband vorwärts bewegt werden kann, wo er durch Bestrahlung mit Röntgenstrahlen, Gammastrahlen oder Magnetstrahlen laufend durchleuchtet wird, wenn er durch ein nichtzerstörerisches Testsystem hindurchtritt, mit dem Ziel, unerwünschte Fremdkörper, sofern vorhanden, in Form von Knochenresten, Nadelteilen etc. zu erkennen, wonach der Artikel an eine Arbeitsstation weitergeleitet wird, wo die Fremdkörper entfernt werden können, wenn der Ort des Fremdkörpers oder der Fremdkörper, sofern vorhanden, einer Bedienperson auf einem Bildschirm angezeigt werden kann, **dadurch gekennzeichnet, dass** das Schirmbild (8) aus der Durchleuchtung auf einem Bildschirm (9) projiziert wird, den die Bedienperson (6) vor ihren Augen trägt, so dass das Schirmbild (8) in Übereinstimmung mit dem Artikel (1) gebracht werden kann, wodurch genau angezeigt wird, wo sich Fremdkörper (4), sofern vorhanden, in dem Artikel (1) befinden.

2. Verfahren nach Anspruch 1, **dadurch gekennzeichnet, dass** die Ausstattung (10) für die Projektion des Schirmbilds (8) und das Verfahren zum Steuern als Teil des Systems und des Herausschneidens in bekannten Schlachtlinien beinhaltet sind.

3. Verfahren nach den Ansprüchen 1 und 2, **dadurch gekennzeichnet, dass** der Bildschirm (8) schwenkbar an einem tragbaren Kopfbekleidungsstück (11) angebracht ist, um das Schirmbild (8) relativ zu der Kopfposition der Bedienperson (6) einzustellen.

## Revendications

1. Procédé de commande et permettant un retrait de parties non voulues d'un article alimentaire, tel que de la viande, de la volaille, du poisson et des légumes, lequel article peut être avancé sur une bande de convoyage, où il est actuellement éclairé par transparence en passant à travers un système de test non destructif par éclairage avec des rayons X, des rayons gamma ou des rayons magnétiques, en vue de détecter des corps étrangers non voulus, le cas échéant, sous la forme de résidus d'os, de parties d'aiguille, etc., après quoi l'article est passé sur une station de travail où les corps étrangers peuvent être retirés, lorsque l'emplacement du corps ou des corps étrangers, le cas échéant, peut être indiqué à un opérateur sur un écran, **caractérisé en ce que** l'image d'écran (8) provenant de l'éclairage par transparence est projetée sur un écran (9) que l'opérateur (6) porte devant ses yeux, de sorte que l'image d'écran (8) peut être mise en concordance avec l'article (1), en indiquant ainsi précisément où des corps étrangers (4), le cas échéant, sont situés dans l'article (1).

2. Procédé selon la revendication 1, **caractérisé en ce que** l'équipement (10) pour la projection de l'image d'écran (8) et le procédé de commande sont inclus dans des lignes d'abattage connues comme une partie du système et la découpe.

3. Procédé selon les revendications 1 et 2, **caractérisé en ce que** l'écran (8) est monté à pivotement sur un vêtement de tête portable (11) pour régler l'image d'écran (8) par rapport à la position de tête de l'opérateur (6).
